# EUROPEAN PATENT SPECIFICATION

(11) **EP 1 309 653 B1**
(45) Date of publication and mention of the grant of the patent: **02.08.2006**
(21) Application number: 00957467.4
(22) Date of filing: 15.08.2000
(51) Int. Cl.: C08K 5/06, C08K 5/17, C08K 5/18, C08K 5/41

(54) **POLYMERIC METHINE ULTRAVIOLET ABSORBERS**
POLYMERE METHIN UV-ABSORBER
ABSORBEURS D'ULTRAVIOLETS EN METHINE POLYMERE

(43) Date of publication of application: 14.05.2003
(73) Proprietor: Milliken & Company, Spartanburg, SC 29303 (US)
(72) Inventor: ZHAO, Xiadong, Edward, Moore, SC 29369 (US); DANIELSON, Todd, D., Moore, SC 29369 (US)
(74) Representative: Klusmann, Peter
(86) International application number: PCT/US2000/022392
(87) International publication number: WO 2002/014418

(56) References cited:
- EP-A2- 0 247 782
- EP-A2- 0 671 501
- US-A- 4 617 373
- US-A- 4 661 566
- US-A- 4 707 537
- US-A- 4 845 187
- US-A- 4 981 516
- J.ORG.CHEM., vol. 54, 1989, pages 145-149, XP002301917
- BOILA-GOECKEL A.; JUNEK H. J.PRAKT.CHEM.-CHEM.ZTG. vol. 342, no. 1, 1999, pages 20 - 28
- DATABASE CROSSFIRE BEILSTEIN [Online] BEILSTEIN INSTITUT ZUR FOERDERUNG DER WISSENSCHAFTEN , FRANKFURT AM MAIN, DE BRN: 6615246, 29 February 2000

## Description

### Technical Field

This invention relates to the use of ultraviolet absorbing methine-based compounds comprising polyoxyalkylene moieties. Such compounds provide excellent, inexpensive, protection from ultraviolet exposure within various media, including, but not limited to, thermoplastics. The presence of polyoxyalkylene chains on the methine backbone permits such an introduction within thermoplastics while simultaneously providing very low degrees of migration from the target substrate. This invention also concerns ultraviolet absorbing thermoplastic compositions and thermoset compositions.

### Background of the Prior Art

Ultraviolet absorber compounds have been utilized for a number of protective applications, including within compositions for covering skin, on and within apparel and other types of textiles, within transparent plastic containers, and the like, to combat the harmful and degradable effects of certain wavelengths of light in the UV spectrum. The best known UV absorbers are benzotriazoles, available from Ciba-Geigy under the tradename Tinuvin®, and benzophenones, available from Cytec Industries under the trademark Cyasorb^{™}. Such compounds are highly effective in their UV absorber capacity; however, they are quite costly, can prove difficult to incorporate within different target media, and tend to migrate from within certain types of media (such as plastics). Furthermore, these two well known types of UV absorbers present handling difficulties in that they are generally produced and utilized in powder form and have relatively low melting points. Particularly, within plastic media, the powder form of these compounds is problematic; a liquid is much easier to handle, does not require melting, and provides more effective and thorough mixing throughout the target plastic. Additionally, these previously utilized UV absorbers provide UV protection over a relatively narrow range of wavelengths (λₘₐₓ from about 290 to about 340 nm for benzotriazoles; from 260 to 300 nm for benzophenones), which ultimately leaves a potentially damaging range of unprotected UV exposure (to about 400 nm). Thus, there exists a need to provide a highly effective, liquid ultraviolet absorber which exhibits a versatility to be incorporated within or applied to different and various media and substrates and which, alternatively, can provide protection over the range of wavelengths in the UV spectrum of from about 290 to about 400 nm (in order to provide the best overall protection from possible harm and/or degradation associated with UV exposure).

Methine-based compounds, in particular certain malonate derivatives, as in European Patent Abstract 350-386-A, to L'Oreal SA, are useful as UV absorbers in cosmetic sunscreen compositions, are generally inexpensive to make, and provide UV protection in the spectrum from about 280 to about 360 nm. However, such compounds are highly soluble in organic solvents and would therefore easily migrate from solid compositions, such as plastics, upon introduction therein. Thus, although the utilization of an effective UV absorber, such as a malonate derivative, within plastics, may be highly desirable, such has never been taught nor fairly suggested within the prior UV absorber art due to the great difficulty in producing such a stable, and thus highly effective, UV absorbing composition from such a methine-based source. There exists a need then to produce an inexpensive UV absorber, preferably methine-based, which possesses the requisite ability to remain within media such as thermoplastics and the like (as noted above), and thus provide necessary and desirable protection from degradation due to UV exposure.

It has now been found that through the addition of polyoxyalkylene chains onto a methine-based ultraviolet absorber compound, greater versatility of potential uses for the new UV absorber is provided. Therefore, it has been found that such polyoxyalkylenated methine-based compounds provide a UV absorber which is highly effective in filtering harmful UV-A and UV-B rays over a broad spectrum (λₘₐₓ from about 320 to about 400 nm, more preferably from about 350 to 390 nm). Furthermore, it has been found that in combination with a benzotriazole and/or a hydroxybenzophenone, or other similar type of UV absorber compound, the resultant composition is accorded protection from a great amount of potentially damaging UV radiation (from approximately 250 to about 400 nm). Additionally, such a combination is highly stable within the target media, and thus provides long-term protection to the desired sample stored within the treated plastic article.

Although some interest has been demonstrated within the area of methine-based UV absorber compounds *(i.e.,* L'Oreal's malonate derivatives), to date there has been no disclosure or fair suggestion regarding the utilization of the polyoxyalkylenated derivatives of such UV absorbers in that capacity within certain media (such as, for example, plastics).

In particular, no disclosures exist concerning methine-based polyalkylenated UV absorber compound which provide an effective protected range of wavelengths from UV exposure of from about 320 to 400 nm. There is thus a great need within the UV absorber market, and most particularly within the transparent plastic film and container markets (for storing and protecting food, pills, and the like) for such types of improvements associated with relatively inexpensive materials and processes provided by the polyoxyalkylenated methine-based UV absorber compounds. EP-A-0 247 782 concerns a process for coloring polyester shaped articles comprising coloring a polyester resin article with a coloring agent of the formula R-(polymeric constituent-X)ₙ, wherein R is an organic dyestuff radical, the polymeric constituent is a polyalkylene oxide or a copolymer thereof and having a molecular weight of 132-500, n is an integer of 1-12 and X is selected from -OH, -NH₂, -SH, -OCOR₁, -CO₂H, -CO₂R₁, -SO₃H, -SO₃R₁ phosphate, phosphonite, urea, urethane, alkoxyl and alkoxide (with R₁ representing a C₁₋₆-alkyl group), and grafting the coloring agent to the article by heating the article to a temperature below its melting temperature but to a sufficiently high temperature and for a sufficiently long period of time to achieve grafting of the coloring agent to the article.

US 4,707,537 discloses polyester compositions suitable for the manufacture of beverage bottles and having condensation reacted therein at least one UV absorbing compound, which is a compound of the formula 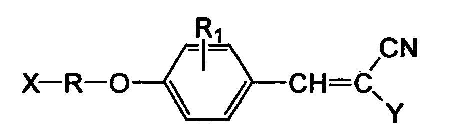 wherein X is hydroxyl, carboxy, optionally substituted carbalkoxy or acyloxy, Y is cyano, optionally substituted carbamyl, aryl, acyl, optionally substituted alkylsulfonyl or optionally substituted phenylsulfonyl, R is selected from -alkylene-O-alkylene-, -alkylene-S-alkylene-, -alkylene-N(-SO₂alkyl)-alkylene- or alkylene optionally substituted with X, and R₁ is hydrogen or 1-3 groups selected from alkyl, alkoxy and halogen.

### Objects of the Invention

It is therefore an object of this invention to provide a novel technique involving a polymeric UV absorber used as an effective UV filtering compound within a suitable composition for protection against potentially harmful ultraviolet rays. It is yet another object of this invention to provide an effective UV absorbing composition or article.

### Description of the Invention

The present invention thus provides the use of a methine-based UV absorber compound of the Formula (I) 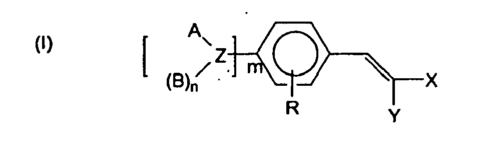 wherein
- R: is selected from C₁₋₂₀-alkyl, halogen, hydroxyl, hydrogen, cyano, sulfonyl, sulfo, sulfato, aryl, nitro, carboxyl, and C₁₋₂₀-alkoxy;
- X, Y: are the same or different and are selected from C(O)OR, C(O)R, C₁₋₂₀-alkyl, and C₁₋₂₀-alkoxy, and R is defined as above;
- Z: is a linking group selected from N, O, and S;
- m: is 1, 2, or 3;
- A: is represented by the Formula (II)

(II) [Alkyleneoxy constituent]_{z}R'

wherein
Alkyleneoxy constituent is C₂₋₂₀-alkylenoxy,
R' is selected from hydrogen, C₁₋₂₀-alkoxy, C₁₋₂₀₋alkyl, and C₁₋₂₀-esters; and
z is in the range of 2-80;
B is selected from hydrogen and A; and
n is either 0 or 1, wherein n = 1 if Z is N, and n = 0 if Z is either O or S;
and having a UV absorption maximum of 320-400 mm, as measured in a polyethylene terephthalate plaque, as a UV filtering compound in polyolefins, halogenated polymers, polyamides, or any mixtures thereof, or in a thermoset component.

Also provided is a thermoplastic composition comprising at least one thermoplastic component selected from a polyolefin, a halogenated polymer, a polyamide, and any mixtures thereof, and a methine-based UV absorber compound having a UV absorption maximum of 320-400 mm, as measured in a polyethylene terephthalate plaque, which is a compound of the Formula (I) as defined above. Preferably, the thermoplastic is a polyolefin, such as polyethylene, polypropylene, polybutylene or any mixture thereof, and more preferably polypropylene.

Furthermore, there is provided a thermoset composition comprising at least one thermoset component and a methine-based UV absorber compound having a UV absorption maximum of 320-400 mm, as measured in a polyethylene terephthalate plaque, which is a compound of the Formula (I) as defined above. Preferably, the thermoset is polyurethane.

In a preferred embodiment, the methine-based UV absorber compound of the Formula (I) is used and is present in the compositions of the invention together with at least one other UV absorber compound, more preferably a UV absorber compound selected from a benzotriazole compound and a hydroxybenzophenone compound.

Accordingly, this invention includes a methine-based ultraviolet absorber compound wherein said compound comprises at least one polyoxyalkylene chain. More specifically, the inventive compound is represented by Formula (I) as defined above.

Preferably, the R group for Formula (I) above is hydrogen, hydroxyl, or C₁₋₁₀ alkyl; most preferably R is hydrogen or hydroxyl. Preferably, X and Y are C(O)OR, where R is C₁₋₁₀ alkyl. Preferably, Alkylene constituent is either oxyethylene, oxypropylene, or oxybutylene, with oxyethylene and oxypropylene most preferred; R' is preferably hydrogen; and Z is preferably 2 to 80, more preferably 3 to 50, and most preferably 5 to 20.

The proper amount utilized in the various compositions and applications is highly dependent on each of those separate possibilities. Thus, in plastics, for example, the inventive UV absorber is added in an amount of 0.001 to 1.5% by weight of the total plastic composition, preferably 0.01 to 1.0%, and most preferably 0.1 to 0.5%.

### Description of the Preferred Embodiments

The general methods of making and utilizing the preferred UV absorbers are as follows:

### UV Absorber Production

The general method of forming the UV absorbing compounds basically entails the reaction of polyalkylenated aniline or resorcinol or similar compound with a malonate, ester, or other similar from carboxyl-group containing compound. The specifically preferred methods (and the preferred compounds as well) are thus provided below:

### EXAMPLE 1

One thousand parts of *p*-formyl-*N,N*-polyoxyethyleneaniline (7 moles EO) were mixed with 124 parts of diethyl malonate and 30 parts of ammonium carbonate. The mixture was then heated between 70 and 75°C for 10 hours. The reaction was monitored by the UV-Vis spectra of the mixture. When the reaction was completed, as indicated by the presence of an absorption maximum at 377 nm (A/gl=20.1), the product was then further stripped under reduced pressure to yield the final product.

### EXAMPLE 2

One-hundred parts of *p*-formyl-*N,N*-polyoxyethylene-polyoxypropylenemiline (7 moles EO and 15 moles PO) were mixed with 24 parts of diethyl malonate, 4.4 parts of morpholine, and 3.3 parts of acetic acid. The mixture was then heated to between 80 and 85°C for 3 hours. The reaction was monitored by the UV-Vis spectra of the mixture. When the reaction was completed, as indicated by the presence of an absorption maximum at 377 nm (A/gl=22.0), the product was further stripped under reduced pressure to yield the final product.

### EXAMPLE 3

Two hundred and fifty parts of *p*-formyl*-N,N*-polyoxyethylene-polyoxypropylene-toluidine (16 moles EO and 10 moles PO) were mixed with 43 parts of diethyl malonate, 7.9 parts of morpholine, and 5.7 parts of acetic acid. The mixture was then heated to between 80 and 85°C for 3 hours. The reaction was monitored by the UV-Vis spectra of the mixture. When the reaction was completed, as indicated by the presence of an absorption maximum at 383.5 nm (A/gl=17.5), the product was further stripped under reduced pressure to yield the final product.

### EXAMPLE 4

Five hundred and twenty parts of *p*-formyl-*N,N*-polyoxyethylene-polyoarypropyleneaniline (7 moles EO and 15 moles PO) were mixed with 78 parts of ethylacetoacetate, 24 parts of morpholine, and 70 parts of acetic acid. The mixture was then heated to between 80 and 85°C for 3 hours. The reaction was monitored by the UV-Vis spectra of the mixture. When the reaction was completed, as indicated by the presence of an absorption maximum at 387.5 nm (A/gl=14.7), the product was further shipped under reduced pressure to yield the final product.

### EXAMPLE 5

Five hundred and forty parts of 2,4-polyoxyalkylene-benzaldehyde (10 moles EO and 10 moles PO) were mixed with one hundred and five parts of diethyl malonate, twenty parts of morpholine, and fourteen parts of acetic acid. The mixture was heated to 95°C. The reaction was monitored by the UV-Vis spectra of the mixture. When the reaction was completed, the product was washed with hot water and then dehydrated to afford the final product exhibiting an absorption maximum at 358 nm (A/gl=12.5).

### EXAMPLE 6

Five hundred parts of the product from EXAMPLE 3, above, were mixed with fifty-five parts of dodecyl succinic anhydride and one part of 1-methyl-imidazole. The mixture was heated at 90°C for four hours under a nitrogen atmosphere. The mixture was further stripped at reduced pressure to afford the final product exhibiting an absorption maximum at 383 nm (A/gl=15.7).

### EXAMPLE 7

One hundred parts of p-formyl N,N polyoxyethylene (8)-polyoxypropylene(2)-*m*-methyl-aniline were mixed with thirty-two parts of diethyl malonate, ten parts of ammonium acetate. The mixture was heated between 80 to 85 °C. The reaction was monitored by the UV-Vis spectra of the mixture. When the reaction was completed, the product was washed twice with equal amount of hot water. The product was further dehydrated under reduced pressure to give the final product (absorption maximum at 383.5 nm; A/gl = 35.0).

### Thermoplastic Composition Formation

The UV absorber was introduced within an injection molding operation for a polyolefin thermoplastic, for instance polypropylene. The liquid absorber was added by way of a positive displacement or transfer pump, either continuously or, preferably, intermittently into an injection molding machine (such as an Arburg Molder). At the same time, the polypropylene resin (in pellet form) was fed into the throat of the molding machine by way of a screw which works in concert with the transfer pump metering the liquid absorber. The rotating screw drew the resin pellets into the feed throat while the pump activated introduction of the liquid absorber into the same area of the machine (in this manner a consistent ratio of pellets to absorber was permitted). At that point, the mix of absorber and pellets was gravity fed into a mixing chamber within the molding machine. In the feed section, melting was accomplished through the utilization of a heated (heat transferred from the barrel of the machine) screw extruder which rotated. The rotating screw also effectuated thorough mixing of the absorber and the molten resin together producing a uniform plastic melt which was then injected into a mold in order to form the desired thermoplastic article.

Testing for absorption improvements and other important criteria was accomplished through the formation of plaques of treated polyethylene terephthalate (PET) (and other types of) resins. These plaques were formed through the process outlined above with the specific compositions listed below in Table 1 for absorbers and PET resins.

**TABLE 1**

| *PET Plaques Produced for Further Testing* | |
|---|---|
| Plaque # | UV Absorber (from Example #) |
| A | 3 |
| B | 2 |
| C | 7 |

The plaques noted above were formed with amounts of UV absorber from 200 to 400 ppm and were 1.3 (50 mils) in thickness. Testing was then performed on the resultant plaques, as noted below.

### Testing for UV properties of the Plastic Compositions

Plaque A was tested for a number of criteria. First, the UV absorption spectrum was taken, including upon introduction of different amounts of the inventive absorber during plaque production. The plaque was exposed to a Xenon Arc lamp source (to simulate sunlight in a broad spectrum) for periods of 10, 20, and 40 hours and subsequently tested after each interval for any change in UV absorption. Measurements were also made prior to exposure to determine the change in lightfastness after the specific exposure intervals. The test results, in correlation to the times exposed and the amounts of UV absorber present are listed below.

Plaques A and C were tested for lightfastness at a loading of about 700 ppm within the polyethylene terephthalate resins. This loading permitted a comparable color loading measurement with a comparative plaque produced from PET with 500 ppm of Tinuvin® 234. Starting at a similar color loading, the two plaques were then measured for lightfastness (K/S) at the same wavelength (here 370 nm) at exposure times of 0, 10 hours, 20 hours, and 40 hours in the presence of a Xenon Arc lamp (to simulate sunlight). Good lightfastness is exhibited through similar K/S measurements over time. The results of this comparison are as follows:

**TABLE 2**

| *Lightfastness Measurements of PET Plaques* | | |
|---|---|---|
| Plaque | Exposure Time (Hours) | K/S Value |
| A | 0 | 5.735 |
| A | 10 | 6.162 |
| A | 20 | 6.100 |
| A | 40 | 5.888 |
| C | 0 | 8.669 |
| C | 10 | 8.643 |
| C | 20 | 8.634 |
| C | 40 | 8.626 |
| Comparative | 0 | 6.080 |
| Comparative | 10 | 6.307 |
| Comparative | 20 | 6.250 |
| Comparative | 40 | 6.233 |

As these measurements indicate, the K/S values for each plaque over time are the same within statistical error. Thus, the inventive UV absorber performs comparably with the commercially available product

### Transmission Data for Polypropylene Resins

In order to compare the percent transmittance of UV light through two different polypropylene resin plaques in accordance with the following Table:

**TABLE 3**

| *Polypropylene Plaques Produced for Further %Transmittance Testing* | | |
|---|---|---|
| Plaque # | UV Absorber | Resin |
| X (Comparative) | 80 ppm Tinuvin® 326 plus | |
| | 20 ppm of Cyasorb® UV-531 | Polypropylene |
| D | 200 ppm of EXAMPLE 1, above, | |
| | plus 20 ppm of Cyasorb® UV-531 | Polypropylene |

These plaques were formulated to possess similar color values. The transmittance spectra of both plaques were measured from 290 nm to 425 nm in 5 nm increments. The results are as follows:

**TABLE 4**

| *% Transmission of UV Light Through Polypropylene Resin* | | |
|---|---|---|
| | Plaques Tested | |
| Wavelength (nm) | X | D |
| 290 | 65.600 | 51.475 |
| 295 | 67.300 | 54.760 |
| 300 . | 65.945 | 54.000 |
| 305 | 65.750 | 52.795 |
| 310 | 65.425 | 51.785 |
| 315 | 65.340 | 50.775 |
| 320 | 66.275 | 50.130 |
| 325 | 67.010 | 49.915 |
| 330 | 68.800 | 50.555 |
| 335 | 68.555 | 49.495 |
| 340 | 68.000 | 47.885 |
| 345 | 67.235 | 45.785 |
| 350 | 67.455 | 44.840 |
| 355 | 67.315 | 44.220 |
| 360 | 67.475 | 43.100 |
| 365 | 69.210 | 42.255 |
| 370 | 70.865 | 42.445 |
| 375 | 72.555 | 42.975 |
| 380 | 74.415 | 43.930 |
| 385 | 77.375 | 46.225 |
| 390 | 79.995 | 49.195 |
| 395 | 82.955 | 53.595 |
| 400 | 84.050 | 57.275 |
| 405 | 85.070 | 62.115 |
| 410 | 86.045 | 67.355 |
| 415 | 86.010 | 72.230 |
| 420 | 86.555 | 76.000 |
| 425 | 86.585 | 79.620 |

Since the lower % transmission represents better performance, it is evident that over the wide range of wavelengths, the inventive UV absorber, in conjunction with a small amount of commercially available UV absorber, provides greater overall protection for the target polypropylene resin.

### Transmission Data for PET Resins

In order to compare the percent transmittance of UV light through four different PET resin plaques in accordance with the following Table:

**TABLE 5**

| *PET Plaques Produced for Further % Transmittance Testing* | | |
|---|---|---|
| Plaque # | UV Absorber | Resin |
| Y (Comparative) | 80 ppm Tinuvin® 326 plus | |
| | 20 ppm of Cyasorb® UV-531 | PET |
| E | 200 ppm of EXAMPLE 1, above, | |
| | plus 20 ppm of Cyasorb® UV-531 | PET |
| Z (Comparative) | 400.ppm Tinuvin® 326 plus | |
| | 100 ppm of Cyasorb® UV-531 | PET |
| F | 1,000 ppm of EXAMPLE 1, above, | |
| | plus 100 ppm of Cyasorb® UV-531 | PET |

These plaques were formulated to possess similar color values. The transmittance spectra of both plaques were measured from 290 nm to 425 nm in 5 nm increments. The results are as follows:

**TABLE 6**

| *% Transmission of UV Light Through PET Resin* | | | | |
|---|---|---|---|---|
| | Plaques Tested | | | |
| Wavelength (nm) | Y | E | Z | F |
| 290 | 0.300 | .0205 | 0.150 | 0.010 |
| 295 | 0.375 | 0.175 | 0.105 | 0.110 |
| 300 | 0.175 | 0.310 | 0.220 | 0.055 |
| 305 | 0.245 | 0.335 | 0.210 | 0.035 |
| 310 | 0.280 | 0.330 | 0.230 | 0.025 |
| 315 | 0.640 | 0.570 | 0.225 | 0.285 |
| 320 | 1.765 | 2.160 | 0.605 | 0.800 |
| 325 | 7.815 | 13.370 | 1.305 | 3.935 |
| 330 | 14.245 , | 24.530 | 2.300 | 6.525 |
| 335 | 17.745 | 30.095 | 2.920 | 6.970 |
| 340 | 18.795 | 32.665 | 2.820 | 6.890 |
| 345 | 19.470 | 35.030 | 2.715 | 6.700 |
| 350 | 20.375 | 37.260 | 2.715 | 6.480 |
| 355 | 21.505 | 38.840 | 2.910 | 6.000 |
| 360 | 23.300 | 39.505 | 3.310 | 5.405 |
| 365 | 26.655 | 41.000 | 4.305 | 4.795 |
| 370 | 30.655 | 41.490 | 6.100 | 4.105 |
| 375 | 36.225 | 41.615 | 8.920 | 3.530 |
| 380 | 41.945 | 41.700 | 13.505 | 3.190 |
| 385 | 50.240 | 42.415 | 20.970 | 3.170 |
| 390 | 58.530 | 43.065 | 31.450 | 3.390 |
| 395 | 68.200 | 45.990 | 46.625 | 4.030 |
| 400 | 74.875 | 48.670 | 60.230 | 5.260 |
| 405 | 80.495 | 53.250 | 72.520 | 8.280 |
| 410 | 83.975 | 59.560 | 79.980 | 13.305 |
| 415 | 86.210 | 66.115 | 83.880 | 21.795 |
| 420 | 86.590 | 71.715 | 85.765 | 32.625 |
| 425 | 87.105 | 77.230 | 86.695 | 46.075 |

Since the lower % transmission represents better performance, it is evident that over the wide range of wavelengths, the methine-based UV absorber, in conjunction with a small amount of commercially available UV absorber, provides greater overall protection for the target PET resin.

### Screw Slippage Testing

The final test was a Screw Slippage Out-put analysis within an extrusion machine. The machine used was a Killion extruder with a 1 inch diameter screw and a 32:1 length:diameter ratio. The time to extrude 1000 g of resin was recorded. The out-put factor for each resin composition was measured as the ratio of time required to extrude 1000 g of treated samples in relation to the pure control sample. An out-put factor at or near 1.00 (either above or below) indicated very little screw slippage and thus a highly favorable result. The composition of the formulations used for Plaques A and B were tested versus a control resin of polyethylene terephthalate resin alone. These inventive compositions both exhibited excellent screw slippage rates of 1.01, nearly the same as for the resin itself.

### Thermoset Composition Formation

The UV absorber was added to the following standard polyurethane foam precursor compositions, one including the UV absorber from Example 3, and one from Example 4 (a control with no UV absorber was also produced with the same formulation):

**COMPOSITION**

| Component | Amount |
|---|---|
| Arcol® F3020 (Aroco) (polyol) | 100.00 g |
| water | 4.52 mL |
| Dabco® 33 LV (Air Products) | 0.15 mL |
| L-520 silicon | 1.50 mL |
| T-10 catalyst | 0.32 mL |
| Toluenediisocyanate (Bayer) | 49.0 mL |
| UV Absorber | 0.50 g |

The compositions were then processed to form foams upon introduction of the water to generate carbon dioxide when reacted with the isocyanate. The resultant foams were each cured for three minutes at 160°C, allowed to cool, and then cut open. There was no measurable difference in bun height or in rise time between the resultant foams containing the methine-based UV absorbers and the control foam product.

## Claims

1. Use of a methine-based UV absorber compound of the Formula (I) 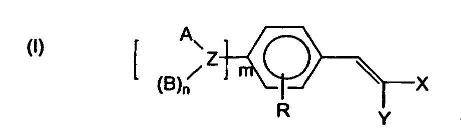 wherein
R is selected from C₁₋₂₀-alkyl, halogen, hydroxyl, hydrogen, cyano, sulfonyl, sulfo, sulfato, aryl, nitro, carboxyl, and C₁₋₂₀-alkoxy;
X, Y are the same or different and are selected from C(O)OR, C(O)R, C₁₋₂₀-alkyl, and C₁₋₂₀-alkoxy, and R is defined as above;
Z is a linking group selected from N, 0, and S;
m is 1, 2, or 3;
A is represented by the Formula (II)
(II) [Alkyleneoxy constituent]_{z}R'
wherein
Alkyleneoxy constituent is C₂₋₂₀-alkylenoxy,
R' is selected from hydrogen, C₁₋₂₀-alkoxy, C₁₋₂₀₋alkyl, and C₁₋₂₀-esters; and
z is in the range of 2-80;
B is selected from hydrogen and A; and
n is either 0 or 1, wherein n = 1 if Z is N, and n = 0 if Z is either O or S;
and having a UV absorption maximum of 320-400 mm, as measured in a polyethylene terephthalate plaque, as a UV filtering compound in polyolefins, halogenated polymers, polyamides, or any mixtures thereof, or in a thermoset component.

2. The use of claim 1, wherein the methine-based UV absorber compound of the Formula (I) is used together with at least one other UV absorber compound.

3. The use of claim 2, wherein the at least one other UV absorber compound is selected from a benzotriazole compound and a hydroxybenzophenone compound.

4. A thermoplastic composition comprising at least one thermoplastic component selected from a polyolefin, a halogenated polymer, a polyamide, and any mixtures thereof, and a methine-based UV absorber compound having a UV absorption maximum of 320-400 mm, as measured in a polyethylene terephthalate plaque, which is a compound of the Formula (I) 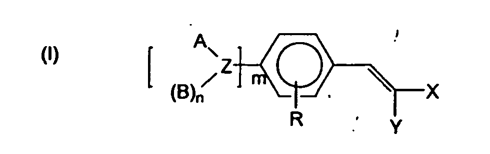 wherein
R is selected from C₁₋₂₀-alkyl, halogen, hydroxyl, hydrogen, cyano, sulfonyl, sulfo, sulfato, aryl, nitro, carboxyl, and C₁₋₂₀-alkoxy;
X, Y are the same or different and are selected from C(O)OR, C(O)R, C₁₋₂₀-alkyl, and C₁₋₂₀-alkoxy, and R is defined as above;
Z is a linking group selected from N, O, and S;
m is 1, 2, or 3;
A is represented by the Formula (II)
(II) [Alkyleneoxy constituent]_{Z}R'
wherein
Alkyleneoxy constituent is C₂₋₂₀-alkylenoxy,
R' is selected from hydrogen, C₁₋₂₀-alkoxy, C₁₋₂₀₋alkyl, and C₁₋₂₀-esters; and
z is in the range of 2-80;
B is selected from hydrogen and A; and
n is either 0 or 1, wherein n = 1 if Z is N, and n = 0 if Z is either O or S.

5. The thermoplastic composition of Claim 4, wherein the thermoplastic is a polyolefin.

6. The thermoplastic composition of Claim 5, wherein the polyolefin is selected from polyethylene, polypropylene, polybutylene, and any mixtures thereof.

7. The thermoplastic composition of Claim 6, wherein the polyolefin is polypropylene.

8. A thermoset composition comprising at least one thermoset component and a methine-based UV absorber compound having a UV absorption maximum of 320-400 mm, as measured in a polyethylene terephthalate plaque, which is a compound of the Formula (I) 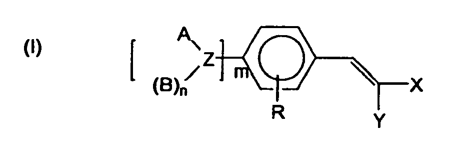 wherein
R is selected from C₁₋₂₀-alkyl, halogen, hydroxyl, hydrogen, cyano, sulfonyl, sulfo, sulfato, aryl, nitro, carboxyl, and C₁₋₂₀-alkoxy;
X,Y are the same or different and are selected from C(O)OR, C(O)R, C₁₋₂₀-alkyl, and C₁₋₂₀-alkoxy, and R is defined as above;
Z is a linking group selected from N, O, and S;
m is 1, 2, or 3;
A is represented by the Formula (II)
(II) [Alkyleneoxy constituent]_{Z}R'
wherein
Alkyleneoxy constituent is C₂₋₂₀-alkylenoxy,
R' is selected from hydrogen, C₁₋₂₀-alkoxy, C₁₋₂₀₋alkyl, and C₁₋₂₀-esters; and
z is in the range of 2-80;
B is selected from hydrogen and A; and
n is either 0 or 1, wherein n = 1 if Z is N, and n = 0 if Z is either O or S.

9. The thermoset composition of Claim 8, wherein the thermoset is polyurethane.

10. The composition of any of claims 4-9, which further comprises at least one other UV absorber compound.

11. The composition of claim 10, wherein the at least one other UV absorber compound is selected from a benzotriazole compound and a hydroxybenzophenone compound.

## Patentansprüche

1. Verwendung einer UV-Absorber-Verbindung auf Methin-Basis der Formel (I) 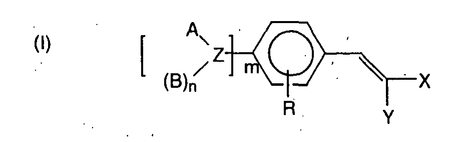 worin
R aus C₁₋₂₀-Alkyl, Wasserstoff, Cyano, Sulfonyl, Sulfo, Sulfat, Aryl, Nitro, Carboxyl und C₁₋₂₀₋Alkoxy ausgewählt wird;
X, Y gleich oder verschieden sind und aus C(O)OR, C(O)R, C₁₋₂₀-Alkyl und C₁₋₂₀-Alkoxy ausgewählt sind und R wie oben definiert ist;
Z eine Verknüpfungsgruppe ist, die aus N, O und S ausgewählt wird;
m 1, 2 oder 3 ist;
A durch die Formel (II) dargestellt wird
(II) [Alkylenoxy-Bestandteil]_{z}R'
worin
der Alkylenoxy-Bestandteil C₂₋₂₀-Alkylenoxy ist,
R' aus Wasserstoff, C₁₋₂₀-Alkoxy, C₁₋₂₀-Alkyl und C₁₋₂₀-Estern ausgewählt wird und
Z im Bereich von 2 bis 80 liegt;
B aus Wasserstoff und A ausgewählt wird; und
n entweder 0 oder 1 ist, worin n = 1 ist, wenn Z N ist, und n = 0 ist, wenn Z O oder S ist;
die ein UV-Absorptionsmaximum von 320-400 nm, gemessen in einer Polyethylenterephthalatplatte, aufweist, als UV-Filterverbindung in Polyolefinen, halogenierten Polymeren, Polyamiden oder beliebigen Mischungen daraus, oder in einer duroplastischen Komponente.

2. Verwendung gemäß Anspruch 1, worin die UV-Absorberverbindung auf Methin-Basis der Formel (I) zusammen mit mindestens einer weiteren UV-Absorberverbindung verwendet wird.

3. Verwendung gemäß Anspruch 2, worin die mindestens eine weitere UV-Absorberverbindung aus einer Benzotriazolverbindung und einer Hydroxybenzophenonverbindung ausgewählt wird.

4. Thermoplastische Zusammensetzung, die mindestens eine thermoplastische Komponente, die aus einem Polyolefin, einem halogenierten Polymer, einem Polyamid und beliebigen Mischungen davon ausgewählt wird, und eine UV-Absorberverbindung auf Methin-Basis mit einem UV-Absorptionsmaximum von 320-400 nm, gemessen in einer Polyethylenterephthalatplatte, die eine Verbindung der Formel (I) ist, umfasst: 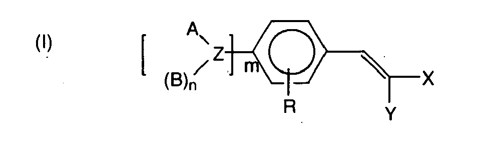 worin
R aus C₁₋₂₀-Alkyl, Wasserstoff, Cyano, Sulfonyl, Sulfo, Sulfat, Aryl, Nitro, Carboxyl und C₁₋₂₀₋Alkoxy ausgewählt wird;
X, Y gleich oder verschieden sind und aus C(O)OR, C(O)R, C₁-₂₀-Alkyl und C₁₋₂₀-Alkoxy ausgewählt werden und R wie oben definiert ist;
Z eine Verknüpfungsgruppe ist, die aus N, O und S ausgewählt wird;
m 1, 2 oder 3 ist;
A durch die Formel (II) dargestellt wird
(II) [Alkylenoxy-Bestandteil]_{z}R'
worin
der Alkylenoxy-Bestandteil C₂₋₂₀-Alkylenoxy ist,
R' aus Wasserstoff, C₁₋₂₀-Alkoxy, C₁₋₂₀-Alkyl und C₁₋₂₀-Estern ausgewählt wird und
Z im Bereich von 2 bis 80 liegt;
B aus Wasserstoff und A ausgewählt wird; und
n entweder 0 oder 1 ist, worin n = 1 ist, wenn Z N ist, und n = 0 ist, wenn Z O oder S ist.

5. Thermoplastische Zusammensetzung gemäß Anspruch 4, worin der Thermoplast ein Polyolefin ist.

6. Thermoplastische Zusammensetzung gemäß Anspruch 5, worin das Polyolefin aus Polyethylen, Polypropylen, Polybutylen und beliebigen Mischungen davon ausgewählt wird.

7. Thermoplastische Zusammensetzung gemäß Anspruch 6, worin das Polyolefin Polypropylen ist.

8. Duroplastische Zusammensetzung, die mindestens eine duroplastische Komponente und eine UV-Absorberverbindung auf Methin-Basis mit einem UV-Absorptionsmaximum von 320-400 nm, gemessen in einer Polyethylenterephthalatplatte, die eine Verbindung der Formel (I) ist, umfasst: 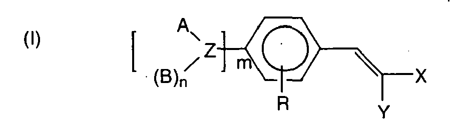 worin
R aus C₁₋₂₀-Alkyl, Wasserstoff, Cyano, Sulfonyl, Sulfo, Sulfat, Aryl, Nitro, Carboxyl und C₁₋₂₀₋Alkoxy ausgewählt wird;
X, Y gleich oder verschieden sind und aus C(O)OR, C(O)R, C₁₋₂₀-Alkyl und C₁₋₂₀-Alkoxy ausgewählt sind und R wie oben definiert ist;
Z eine Verknüpfungsgruppe ist, die aus N, O und S ausgewählt wird;
m 1, 2 oder 3 ist;
A durch die Formel (II) dargestellt wird
(II) [Alkylenoxy-Bestandteil]_{z}R'
worin
der Alkylenoxy-Bestandteil C₂₋₂₀-Alkylenoxy ist,
R' aus Wasserstoff, C₁₋₂₀-Alkoxy, C₁₋₂₀-Alkyl und C₁₋₂₀-Estern ausgewählt wird und
Z im Bereich von 2 bis 80 liegt;
B aus Wasserstoff und A ausgewählt wird; und
n entweder 0 oder 1 ist, worin n = 1 ist, wenn Z N ist, und n = 0 ist, wenn Z O oder S ist.

9. Duroplastische Zusammensetzung gemäß Anspruch 8, worin das Duroplast Polyurethan ist.

10. Zusammensetzung gemäß mindestens einem der Ansprüche 4 bis 9, die ferner mindestens eine weitere UV-Absorberverbindung umfasst.

11. Zusammensetzung gemäß Anspruch 10, worin die mindestens eine weitere UV-Absorberverbindung aus einer Benzotriazolverbindung und einer Hydroxybenzophenonverbindung ausgewählt wird.

## Revendications

1. Utilisation d'un composé à base de méthine absorbant les UV de formule (I) 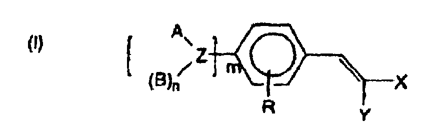 où
R est choisi parmi un groupe alkyle en C_{1 à 20}, un atome d'halogène, un groupe hydroxyle, un atome d'hydrogène, un groupe cyano, sulfonyle, sulfo, sulfato, aryle, nitro, carboxyle, et alkoxy en C_{1 à 20} ;
X, Y sont identiques ou différents et sont choisis parmi C(O)OR, C(O)R, un groupe alkyle en C_{1 à 20}, et alcoxy en C_{1 à 20,} et R est comme défini ci-dessus ;
Z est un groupe liant choisi parmi N, O et S ;
m vaut 1, 2 ou 3 ;
A est représenté par la formule (II)
(II) [constituant alkylèneoxy]_{z}R'
où
le constituant alkylèneoxy est un groupe alkylènoxy en C_{2 à 20},
R' est choisi parmi un atome d'hydrogène, un groupe alcoxy en C_{1 à 20}, alkyle en C_{1 à 20}, et un ester en C_{1 à 20} ; et
z est dans la gamme de 2 à 80 ;
B est choisi parmi un atome d'hydrogène et A ; et
n vaut soit 0 ou 1, où n = 1 si Z est N, et n = 0 si Z est soit O ou S ;
et ayant un maximum d'absorption des UV de 320 à 400 mm, comme mesuré dans une plaque de poly(téréphtalate d'éthylène), en tant que composé filtrant les UV dans les poly(oléfines), les polymères halogénés, les poly(amides), ou tous mélanges de ceux-ci, ou dans un composant thermodurci.

2. Utilisation selon la revendication 1, dans laquelle le composé à base de méthine absorbant les UV de formule (I) est utilisé conjointement avec un autre composé absorbant les UV.

3. Utilisation selon la revendication 2, dans laquelle l'au moins un autre composant absorbant les UV est choisi parmi un composé de benzotriazole et un composé d'hydroxybenzophénone.

4. Composition thermoplastique comprenant au moins un composant thermoplastique choisi parmi une poly(oléfine), un polymère halogéné, un poly(amide), et tous mélanges de ceux-ci, et un composé à base de méthine absorbant les UV ayant un maximum d'absorption du rayonnement UV de 320 à 400 mm comme mesuré dans une plaque de poly(téréphtalate d'éthylène), qui est un composé de formule (I) 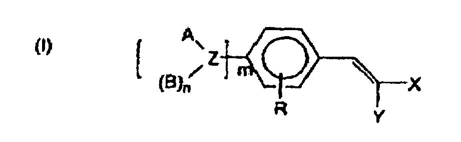 où
R est choisi parmi un groupe alkyle en C_{1 à 20}, un atome d'halogène, un groupe hydroxyle, un atome d'hydrogène, un groupe cyano, sulfonyle, sulfo, sulfato, aryle, nitro, carboxyle, et alkoxy en C_{1 à 20} ;
X, Y sont identiques ou différents et sont choisis parmi C(O)OR, C(O)R, un groupe alkyle en C_{1 à 20,} et alcoxy en C_{1 à 20}, et R est comme défini ci-dessus ;
Z est un groupe liant choisi parmi N, O et S ;
m vaut 1, 2 ou 3 ;
A est représenté par la formule (II)
(III) [constituant alkylèneoxy]_{z}R'
où
le constituant alkylèneoxy est un groupe alkylènoxy en C_{2 à 20},
R' est choisi parmi un atome d'hydrogène, un groupe alcoxy en C_{1 à 20}. alkyle en C_{1 à 20}, et un ester en C_{1 à 20} ; et
z est dans la gamme de 2 à 80 ;
B est choisi parmi un atome d'hydrogène et A ; et
n vaut soit 0 ou 1, où n = 1 si Z est N, et n = 0 si Z est soit O ou S.

5. Composition thermoplastique selon la revendication 4, dans laquelle la thermoplastique est une poly(oléfine).

6. Composition thermoplastique selon la revendication 5, dans laquelle la poly(oléfine) est choisi parmi le poly(éthylène), le poly(propylène), le poly(butylène), et tous mélanges de ceux-ci.

7. Composition thermoplastique selon la revendication 6, dans laquelle la poly(oléfine) est le poly(propylène).

8. Composition thermodurcissable comprenant au moins un composant thermodurcissable et un composé à base de méthine absorbant les UV et ayant un maximum d'absorption des UV de 320 à 400 mm, comme mesuré dans une plaque de poly(téréphtalate d'éthylène), qui est un composé de formule (I) 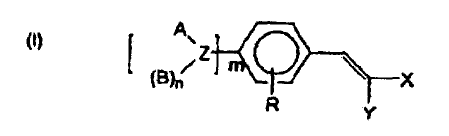 où
R est choisi parmi un groupe alkyle en C_{1 à 20}, un atome d'halogène, un groupe hydroxyle, un atome d'hydrogène, un groupe cyano, sulfonyle, sulfo, sulfato, aryle, nitro, carboxyle, et alkoxy en C_{1 à 20} ;
X, Y sont identiques ou différents et sont choisis parmi C(O)OR, C(O)R, un groupe alkyle en C_{1 à 20}, et alcoxy en C_{1 à 20}, et R est comme défini ci-dessus ;
Z est un groupe liant choisi parmi N, O et S ;
m vaut 1, 2 ou 3 ;
A est représenté par la formule (II)
(II) [constituant alkylèneoxy]_{z}R'
où
le constituant alkylèneoxy est un groupe alkylènoxy en C_{2 à 20},
R' est choisi parmi un atome d'hydrogène, un groupe alcoxy en C_{1 à 20}. alkyle en C_{1 à 20}, et un ester en C_{1 à 20} ; et
z est dans la gamme de 2 à 80 ;
B est choisi parmi un atome d'hydrogène et A ; et
n vaut soit 0 ou 1, où n = 1 si Z est N, et n = 0 si Z est soit O ou S.

9. Composition thermodurcissable selon la revendication 8, dans laquelle le thermodurcissable est le poly(uréthane).

10. Composition selon l'une quelconque des revendications 4 à 9, qui comprend en outre au moins un autre composé absorbant les UV.

11. Composition selon la revendication 10, dans laquelle l'au moins un autre composé absorbant les UV est choisi parmi un composé de benzotriazole et un composé d'hydroxybenzophénone.
